# EUROPÄISCHE PATENTSCHRIFT

(11) **EP 1 415 672 B1**
(45) Veröffentlichungstag und Bekanntmachung des Hinweises auf die Patenterteilung: **10.09.2014**
(21) Anmeldenummer: 03090442.9
(22) Anmeldetag: 19.04.2000
(51) Int. Cl.: A61M 1/10, A61M 1/36, F04D 3/02, F04D 13/06, F04D 15/00, F04D 29/041, F16C 32/04, F16C 39/06, H02K 5/128, H02K 7/09, H02K 7/14, H02K 11/00

(54) **Pumpe mit Drucksensor**
Pump with pressure sensor
Pompe avec capteur de pression

(30) Priorität: 20.04.1999 DE 19918841
(43) Veröffentlichungstag der Anmeldung: 06.05.2004
(62) Teilanmeldung aus: 00927001.8
(73) Patentinhaber: Berlin Heart GmbH, 12247 Berlin (DE); Forschungszentrum Jülich GmbH, 52425 Jülich (DE)
(72) Erfinder: Nüsser, Peter, 13051 Berlin (DE); Müller, Johannes, 10717 Berlin (DE); Peters, Hans-Erhard, 10437 Berlin (DE); Fremerey, Johan K., 53127 Bonn (DE)
(74) Vertreter: Pfenning, Meinig & Partner GbR

(56) Entgegenhaltungen:
- EP-A- 0 856 666
- WO-A1-96/31934
- WO-A1-98/43688
- US-A- 5 211 546
- US-A- 5 385 581

## Beschreibung

Die Erfindung betrifft eine Vorrichtung zur schonenden Förderung von ein- oder mehrphasigen Fluiden gemäß dem Oberbegriff des Anspruches 1.

Insbesondere geringer stabile mehrphasige Fluide, die durch einen Energieeintrag irreversible Veränderungen erfahren können, wie z.B. Emulsionen und Dispersionen, können beim Fördern in entsprechenden Vorrichtungen wie Pumpen nachteiligerweise in instabile Bereiche geraten.

Ein besonders empfindliches Fluidsystem stellt das Blut dar. Diese undurchsichtige rote Körperflüssigkeit der Wirbeltiere zirkuliert in einem in sich geschlossenen Gefäßsystem, wobei rhythmische Kontraktionen des Herzens das Blut in die verschiedenen Gebiete des Organismus hineindrücken. Hierbei transportiert das Blut die Atemgase Sauerstoff und Kohlendioxid sowie Nährstoffe, Stoffwechselprodukte und körpereigene Wirkstoffe. Das Blutgefäßsystem einschließlich des Herzens ist hierbei hermetisch von der Umwelt abgeschirmt, so dass im gesunden Organismus das Blut keine Veränderungen erfährt, wenn es über das Herz durch den Körper gepumpt wird.

Bekannt ist, dass das Blut bei Kontaktierung mit nichtkörpereigenen Materialien oder durch Fremdenergieeinwirkung zur Hämolyse und Thrombenbildung neigt. Thrombenbildung kann für den Organismus tödlich sein, weil sie zu Verstopfungen im weitverzweigten Gefäßsystem führen kann. Hämolyse beschreibt den Zustand, dass über das physiologische Maß hinaus die roten Blutkörperchen innerhalb des Körpers lysiert - zerstört - werden. Die Ursachen für Hämolyse können mechanisch oder metabolischer Art sein. Gesteigerte Hämolyse hat multiple Organschäden zur Folge und kann bis zum Tode des Menschen führen.

Andererseits hat sich gezeigt, dass es prinzipiell möglich ist, unter bestimmten konstruktiven Voraussetzungen, die Pumpleistung des Herzens zu unterstützen bzw. sogar das natürliche Herz durch ein Kunstherz zu ersetzen. Allerdings ist ein Dauerbetrieb von implantierten Herzunterstützungspumpen oder Kunstherzen zur Zeit nur begrenzt möglich, weil die Wechselwirkungen dieser Kunstprodukte mit dem Blut immer noch zu nachteiligen Veränderungen des Blutes führen.

Im bekannten Stand der Technik sind verschiedene Entwicklungsrichtungen von Blutpumpen erkennbar. Herzunterstützungspumpen und Kunstherzen können ausgehend von der geforderten Druckdifferenz und dem Volumenstrom sowohl nach dem Verdrängerprinzip als sogenannte pulsatile Pumpen oder nach dem Turboprinzip als radiale oder axiale Strömungsmaschinen ausgeführt werden. Diese drei genannten Bauarten werden zur Zeit parallel entwickelt. Dabei zeigen die Strömungsmaschinen wegen der hohen Leistungsdichte dieser Maschinenart kleinere Abmessungen als Kolbenmaschinen. Innerhalb der Pumpen, die nach dem Turboprinzip funktionieren, ist die axiale Pumpenvariante in der Regel kleiner als die radiale. Hierbei läßt sich grundsätzlich eine Turbomaschine zu gegebener Druckdifferenz und gegebenem Volumenstrom sehr unterschiedlich sowohl als axiale als auch radiale Pumpe mit sehr unterschiedlichen Drehzahlen ausführen.

Die aus dem Stand der Technik bekannten axialen Blutpumpen bestehen im Wesentlichen aus einem äußeren zylindrischen Rohr, in dem ein Förderteil, das als Rotor eines außen anliegenden Motorstators ausgebildet ist, rotiert und damit das Blut in axialer Richtung bewegt. Die Lagerung des Förderteils stellte ein Problem dar. Eine rein mechanische Lagerung ist hinsichtlich der Blutschädigung und auch der relativ hohen Reibungswerte nachteilig. Auch die bisher beschriebenen Magnetlagerungsvarianten haben zu keiner befriedigenden Lösung geführt.

Aus Kawahito et al.: In Phase 1 Ex Vivo Studies of the Baylor/NASA Axial Flow Ventricular Assist Device, in: Heart Replacement Artificial Heart 5, Seiten 245-252, Springer Verlag Tokyo 1996, Herausgeber T. Akutso und H. Koyagani, ist eine gattungsgemäße axiale Blutpumpe zur Unterstützung eines erkrankten Herzens bekannt, die in den Brustraum eines Patienten implantierbar ist. Die axiale Blutpumpe weist ein rotierendes Laufrad mit einer Beschaufelung auf, das innerhalb eines blutführenden Rohres gelagert und mittels eines Elektromotors angetrieben wird.

Hierzu ist das Laufrad als Rotor des Elektromotors ausgebildet und über in der Beschaufelung angebrachte Magnete mit dem gehäusefesten Stator des Elektromotors gekoppelt. Eine Axial- und Radiallagerung des Rotors erfolgt über eine Spitzenlagerung, bei der der Rotor punktweise an in der Strömung angeordneten Lagerelementen abgestützt wird. Eine derartige Anordnung ist auch aus der US A 4 957 504 bekannt.

Die bekannte Blutpumpe weist den Nachteil auf, dass das geförderte Blut in nicht unerheblichem Ausmaße eine Traumatisierung und Schädigung erfährt. Insbesondere besteht die Gefahr einer Thrombenbildung. Der Grund hierfür liegt im Wesentlichen in der Ausbildung von Totwassergebieten an den Lagern.

Ein weiterer Nachteil besteht zweifelsfrei in der begrenzten Standzeit der mechanischen Lager infolge Verschleißes.

Im US-Patent 4 779 614 wird eine implantierbare axiale Blutpumpe beschrieben, die aus einem äußeren zylindrischen Rohr und einer in diesem Rohr rotierenden Rotornabe zur Blutförderung besteht. Der Rotor ist magnetisch gelagert und trägt gleichzeitig die Rotormagnete des Antriebes und die Laufschaufeln. Der magnetisch gelagerte Rotor bildet mit der am äußeren Rohr befestigten Statorbeschaufelung lange, enge Spalte. Durch die Anordnung von zwei Motor-Stator-Kombinationen jeweils an den Enden der Pumpe soll die radiale Lage des Rotors stabilisiert werden. Die Position in Achsrichtung wird durch ein weiteres Magnetpaar, das auch die Axialkräfte des Rotors aufnehmen soll, stabilisiert. Obwohl ein relativ breiter Ringspalt für die Fluidströmung vorgesehen, ist und mit der magnetischen Lagerung des Rotors wesentliche Entwicklungsziele für implantierbare Blutpumpe bezüglich kompakter Bauweise und Freiheit von Dichtungsund Lagerproblemen verfolgt werden können, weist diese Blutpumpe gravierende Nachteile für die Funktion und den konstruktiven Aufbau der Pumpe auf. Die überlangen engen Spalte zwischen der Rotornabe und den Leitschaufeln am Stator erhöhen die Gefahr der Blutschädigung durch große Geschwindigkeitsgradienten der Spaltströme. Die zur Rotorstabilisierung notwendige Anordnung von zwei Motoren ist konstruktiv aufwendig. Weiterhin ist der Rotor in axialer Richtung nicht formschlüssig gesichert und stellt dadurch ein Restrisiko dar.

In dem US-Patent 5 385 581 ist ebenfalls eine axiale Blutpumpe mit magnetischer Lagerung beschrieben. Die Lagermagneten sind im Rotor und im Statorbereich entgegengesetzt gepolt angeordnet.

Nachteiligerweise führt das zum Stillstand der Pumpe, wenn die Lagerung versagt. Ferner ist es nachteilig, dass kein so genanntes Nachleitgitter vorgesehen ist, das heißt, den gesamten Druck baut das Laufrad auf, und der Restdrall verbleibt in der Strömung.

Eine weitere axiale Blutpumpe mit magnetischer Lagerung ist aus WO 97/49 440 bekannt. Die magnetische Lagerung erfolgt an den konisch gestalteten Läuferenden des Rotors, der das Flügelrad bildet. Den Läuferenden stehen fest angeordnete Polschuhe gegenüber, die den Fluß von Permanentmagneten leiten. Die Lagerung erfordert eine aktive Stabilisierung mit mindestens vier Stabilisatorspulen sowohl in axialer als auch in radialer Richtung. In weiteren Varianten werden Lagerungen mit radial magnetisierten Permanentmagnetringen mit wechselnder Magnetisierungsrichtung vorgeschlagen, die technisch nur schwer beherrschbar sind.

Bekannt ist aus WO 98/11 650 eine weitere axiale Blutpumpe mit einem sogenannten lagerlosen Motor. Der "lagerlose" Motor ist eine Kombination aus Motor und magnetischer Lagerung. Die Lage des Rotors ist bezüglich dreier Freiheitsgrade - Translation in x-Richtung, Verkippen in x- und y-Richtung - passiv durch Permanentmagnete stabilisiert. Die passive Stabilisierung wird durch einen permanentmagnetischen Rotorring realisiert, der statorseitig von einem Weicheisenring umgeben ist. Steuer- und Antriebswicklungen, die mit dem Weicheisenring verbunden sind, gestatten eine Ansteuerung bezüglich dreier Freiheitsgrade. Die geringe Lagersteifigkeit erfordert zusätzliche Maßnahmen. Außerdem ist eine Lagestabilisierung in x- und y-Richtung nötig, was zu einem hohen messtechnischen Aufwand führt und eine hohe Erwärmung der Pumpe durch die aktiven Spulen zur Folge haben kann.

Zur Förderung chemischer Flüssigkeiten ist eine axiale Schraubenpumpe aus EP-A 0 856 666 bekannt. Das Förderteil ist dort zwischen zwei Befestigungsteilen magnetisch gelagert, die unter Belassung eines Ringspaltes in einem rohrförmigen Hohlkörper befestigt sind. Das Förderteil bildet den Rotor eines Motors, dessen Stator außerhalb des rohrförmigen Hohlkörpers angeordnet ist. Die magnetische Lagerung erfolgt in radialer Richtung mit radial magnetisierten Permanentmagneten und, davon weitestgehend entkoppelt, in axialer Richtung. mittels. elektromagnetischer Spulen. Radial magnetisierte Permanentmagnete erfordern eine bestimmte Mindestbaugröße und kleine Luftspalte.

Der Förderspalt darf also nur sehr klein sein, was bei der hier vorliegenden Förderaufgabe (Schraubenpumpen erzeugen einen hohen Druck bei kleinem Fördervolumen) nicht hinderlich, für andere Pumpen, insbesondere Blutpumpen jedoch nicht akzeptabel ist. Außerdem muß die gesamte axiale Steifigkeit, die wegen des Förderdrucks des zu fördernden Mediums sehr hoch gegenüber der radialen Steifigkeit ist, durch die Stabilisierungsspulen aufgebracht werden, was eine bestimmte Stromhöhe voraussetzt, die zu entsprechendem Energiebedarf und zur Erwärmung führt. Die Regelung der axialen Position wird mit zunehmender Stromhöhe zudem träger, so dass die Pumpe für pulsatile Förderaufgaben nur bedingt geeignet ist.

Wichtig bei dem Betrieb der Vorrichtung ist auch eine exakte Erfassung der wichtigsten Strömungsparameter wie Druck, durchströmendes Volumen pro Zeiteinheit etc.

Der Erfindung liegt die Aufgabe zugrunde, eine Vorrichtung zur schonenden Förderung von ein- und mehrphasigen Fluiden zur Verfügung zu stellen, die bei einfachem konstruktivem Aufbau das zu fördernde Fluid in seinen Eigenschaften nicht oder nur unwesentlich verändert, Totwassergebiete und Verwirbelungen des zu fördernden Fluids minimiert und eine pulsierende Förderung ermöglicht. Hierbei soll die Messung wichtiger Strömungsparameter möglichst exakt erfolgen.

Die Lösung der Aufgabe erfolgt erfindungsgemäß mit den kennzeichnenden Merkmalen des Anspruches 1.

Bevorzugte und vorteilhafte Ausgestaltungen der Erfindung sind in den Unteransprüchen angegeben.

Eine weitere Ausgestaltung sieht vor, dass das Förderteil berührungsfrei zwischen den Befestigungselementen, durch je einen Nabenspalt getrennt, mittels sowohl in den Befestigungselementen als auch im Förderteil angeordneten, funktionell zusammenwirkenden, sich mit ihren magnetisch wirksamen Flächen gegenüberstehenden, in axialer Richtung magnetisierten, entgegengesetzt gepolten permanentmagnetischen Lagerelementen gelagert ist. Sensoren zur Positionserfassung und Stabilisatoren zur Positionskorrektur des Förderteiles sind in den Befestigungselementen und an oder in der Wandung des Hohlkörpers angeordnet.

Die erfindungsgemäße Vorrichtung stellt einen einfachen konstruktiven Aufbau zur Verfügung. Die für die magnetische Lagerung erforderlichen permanentmagnetischen Lagerelemente sind zusätzlich zu den permanentmagnetischen Elementen des Motorrotors unmittelbar am Förderteil angeordnet. Die magnetische Lagerung nimmt vorteilhafterweise sowohl die axialen als auch die radialen Kräfte auf. Die Axialstabilisierung stellt eine aktive Regelung der axialen Lage des Förderteiles zur Verfügung, wobei dem Förderteil stirnseitig zugeordnete Ringspulen einen axialen Magnetfluß erzeugen, der den axialen Magnetfluß der permanentmagnetischen Lagerelemente überlagert und der Regelung der axialen Lage dient.

Der notwendigerweise vorhandene Rotorspalt zwischen der Außenseite des Förderteiles und der Innenseite des rohrförmigen Hohlkörpers ist dabei derart auszulegen, dass sowohl die Motorverluste als auch durch den Spalt auftretende Strömungsverluste minimiert werden. Dabei ist zu beachten, dass die auftretenden Motorverluste um so größer sind, je weiter der Motorrotor vom Motorstator entfernt ist. Ein kleiner Rotorspalt ist motorseitig daher als günstig anzusehen. Andererseits führt ein kleiner Rotorspalt jedoch zu großen Reibungsverlusten der Strömung und ist daher strömungstechnisch ungünstig. Ein geeigneter Kompromiß für Blutpumpen liegt beispielsweise in der genannten Rotorspaltbreite von 0,5 bis 2,5 mm.

Eine vorteilhafte Ausbildung der Erfindung besteht darin, dass in die Naben der axialen Blutpumpe und/oder in die Wandung des rohrförmigen Hohlkörpers weitere Sensoren zur Erfassung des momentanen Blut-Volumenstroms und der momentan von der Pumpe erzeugten Druckdifferenz integriert sind. Beide Meßgrößen stehen im Controller der Fördereinrichtung für Soll-Ist-Vergleiche zur Verfügung und eröffnen damit die Möglichkeit für eine Regelung des Fördervorganges im Sinne einer physiologisch optimalen, der natürlichen Herzaktion angepaßten pulsatilen Förderung mittels zeitabhängiger Drehzahländerung des Rotors oder einer im Sinne geringen Energieverbrauchs optimierten pulsatilen Förderung durch die Pumpe, gleichfalls realisiert durch zeitabhängige Drehzahländerung.

In bevorzugter Weise sind die Befestigungselemente als Fluid-Leiteinrichtungen mit Fluidbeschaufelungen ausgebildet. Dadurch werden Strömungsverluste minimiert.

In einer weiteren vorteilhaften Weiterbildung der Erfindung sind an der Stirnseite der Rotornabe Mittel vorgesehen, die im Nabenspalt zwischen Fluid-Leiteinrichtung und Förderteil befindliches Fluid radial nach außen fördern, etwa radiale Beschaufelungen, Rillen, Ausbuchtungen oder ballige Formgebungen.

Eine weitere vorteilhafte Ausgestaltung der Erfindung besteht darin, dass in mindestens einer der Fluid-Leiteinrichtungen eine axial verlaufende Bohrung vorgesehen ist, die von zu förderndem Fluid durchströmt wird und die bewirkt, dass im Nabenspalt zwischen Fluid-Leiteinrichtung und Förderteil befindliches Fluid radial nach außen transportiert wird.
Beide vorgenannten Weiterbildungen beeinflussen die radiale Druckverteilung und generieren Ausgleichsströmungen zur Verhinderung von Totwassergebieten im Nabenspalt zwischen den Stirnseiten von Fluid-Leiteinrichtung und Förderteil.

In einer weiteren Ausbildung der Erfindung weist das Förderteil, insbesondere die Rotornabe in axialem Abstand zwei Beschaufelungen auf. Hierdurch wird ein sogenanntes Tandemgitter gebildet.

Vorteilhafterweise wird dadurch die je Schaufelreihe zu erbringende Druckerhöhung herabgesetzt. Darüber hinaus schränkt diese besondere Ausbildung des Rotors der Fördereinrichtung störende Kippbewegungen desselben zusätzlich ein.

Die Erfindung wird nachfolgend unter Bezugnahme auf die Figuren der Zeichnung beispielhaft näher erläutert.

Es zeigen
- Fig. 1: eine axiale Blutpumpe in Schnittdarstellung,
- Fig. 2: eine axiale Fördervorrichtung mit Magnetlagerung; Axialstabilisierung und Positionssensorik in Längsschnittdarstellung,
- Fig. 2a: eine Schnitt-A-A-Darstellung der axialen Fördervorrichtung gemäß Fig. 2,
- Fig. 2b: eine axiale Fördervorrichtung mit Magnethalterung in Längsschnitt,
- Fig. 2c: eine Schnitt-A-A-Darstellung der axialen Fördervorrichtung gemäß Fig. 2b,
- Fig. 2d: eine axiale Fördervorrichtung mit konischem Förderteil in Längsschnittdarstellung,
- Fig. 3a: eine Magnethalterung für eine axiale Fördervorrichtung,
- Fig. 3b: die Magnethalterung gemäß Fig. 3a im Querschnitt,
- Fig. 4: ein Förderteil mit Doppelbeschaufelung,
- Fig. 5: eine Fluid-Leiteinrichtung mit Positionssensor und permanentmagnetischem Lagerelement,
- Fig. 5a: die Fluid-Leiteinrichtung gemäß Fig. 5 in der Darstellung Schnitt-B-B,
- Fig. 6a: eine schematische Vorderansicht der Stirnseite einer Rotornabe oder Nabe,
- Fig. 6b: eine schematische Vorderansicht der Stirnseite einer weiteren Rotornabe oder Nabe,
- Fig. 6c: eine schematische Vorderansicht der Stirnseite einer Rotornabe oder Nabe mit exzentrischer Erhöhung,
- Fig. 7: eine schematische Schnittdarstellung eines Nabenspaltes, gebildet zwischen Förderteil und Nabe eines Befestigungselementes,
- Fig. 7a: eine schematische Schnittdarstellung eines Nabenspaltes gebildet zwischen Förderteil und Nabe eines Befestigungselementes, und
- Fig. 8: eine schematische Schnittdarstellung durch eine Nabe mit axialer Bohrung.

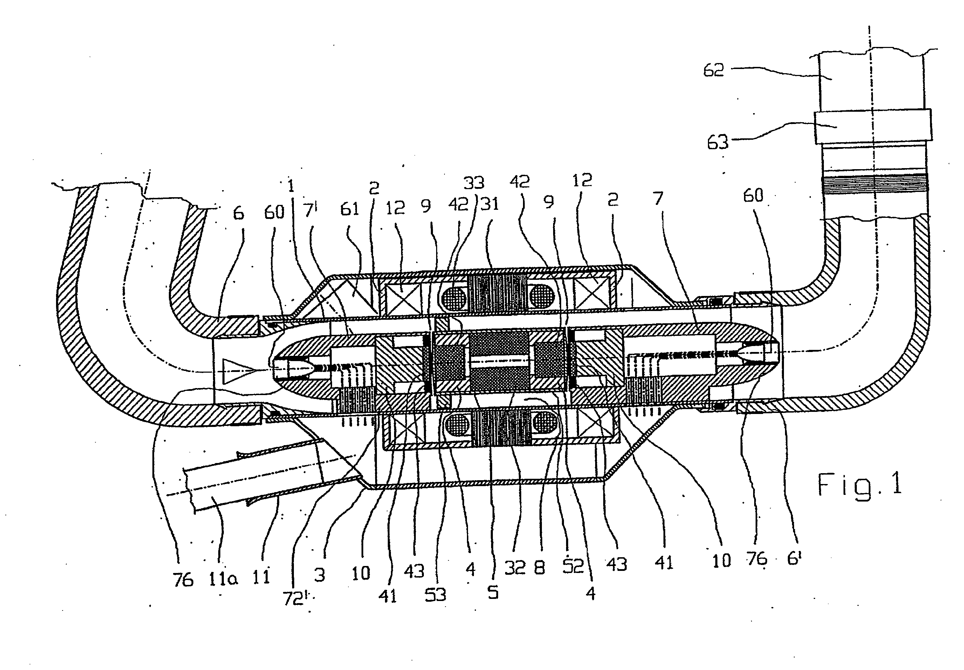

Fig. 1 zeigt eine beispielhafte Ausführung einer erfindungsgemäßen Blutpumpe mit Pumpengehäuse 3 und Stabilisatorgehäuse 2. Außerhalb eines rohrförmigen Hohlkörpers 1, in dem in axialer Richtung das Fluid gefördert wird, ist um den Hohlkörper 1 herum ein Motorstator 31 mit Motorwicklungen 33 angeordnet. Der Motorstator 31 treibt ein Förderteil 5 an, das einen Motorrotor 32 und eine Rotornabe 52 enthält und das im Inneren des rohrförmigen Hohlkörpers 1 gelagert ist. Die Rotornabe 52 weist eine Rotorbeschaufelung 53 auf. In Strömungsrichtung vor und hinter der Rotornabe 52 sind Fluid-Leiteinrichtungen 7 und 7' mit Fluid-Leitbeschaufelungen 72 und 72' an der Innenwand des rohrförmigen Hohlkörpers 1 fixiert. Zwischen den Fluid-Leiteinrichtungen, 7 und 7' und der Rotornabe 52 ist ein so genannter Nabenspalt 9 ausgebildet. Über den Motorstator 31 ist der Motorrotor 32, der mit der Rotornabe 52 kombiniert ist, in Rotation versetzbar.

Beim Betrieb der Blutpumpe wird das aus dem Herzen ausströmende Blut durch einen Krümmer 6 dem Förderteil 5.zugeführt und wird dort mittels der Rotorbeschaufelung 53 in Rotation versetzt, wobei die Rotornabe 52 für strömungsdynamisch günstige Verhältnisse sorgt. Für eine strömungstechnisch vorteilhafte Anströmung der Rotorbeschaufelungen 53 sorgt die stromaufwärts fest mit dem Hohlkörper 1 verbundene Fluid-Leiteinrichtung 7' mit ihren Beschaufelungen 72'. Der Drucksensor 60 erlaubt die Druckmessung im zuströmenden Fluid. Das Förderteil 5 erhält seinen Antrieb in an sich bekannter Weise durch magnetische Kopplung des Motorrotors 32 mit dem Motorstator 31. Eine Bildung von Thromben bei Blut als zu förderndem Medium ist stark minimiert, da aufgrund der Magnetlagerung keine Lagerelemente in der Strömung angeordnet sind, die eine Bildung von Totwassergebieten herbeiführen könnten. Eine Verwirbelung und damit verbundene Strömungsverluste erfolgt lediglich in geringem Maße. Ein Rotorspalt 8 zwischen Rotornabe 52 und Innenwand des Hohlkörpers 1 weist dabei eine Breite auf, die die Strömungsverluste klein hält und gleichzeitig auch die Motorverluste begrenzt, die mit zunehmendem Abstand des Motorrotors 32 vom Motorstator 31 zunehmen. Als besonders günstig hat sich beispielsweise eine Breite des Rotorspaltes 8 zwischen 0,5 und 2,5 mm herausgestellt. Nach Beschleunigung des Fluids durch die Rotorbeschaufelung 53 der Rotornabe 52 und einem damit einhergehenden Druckaufbau wird das Fluid in die Fluid-Leiteinrichtung 7 geleitet, wo es eine Umlenkung in axiale Richtung erfährt und ein weiterer Druckaufbau erfolgt. Durch die. Formgebung der Fluid-Leitbeschaufelung 72 der Fluid-Leiteinrichtung 7 wird sichergestellt, dass die Umlenkung des Fluids in axialer Richtung schonend und ebenfalls im Wesentlichen ohne Verwirbelung erfolgt.

Das Blut verläßt die Blutpumpe über einen Krümmer 6' und strömt in eine Aortenkanüle 62, die am Krümmer 6' mittels eines lösbaren Verbindungselementes 63 befestigt ist. Ein speziell geschirmtes Kabel 11a, das die Versorgungs- und die Signalleitungen für den Motorstator 31, den Axialstabilisator 12 und die Sensorik 60, 61 und 43 enthält, ist über den Kabelstutzen 11 mit der Blutpumpe verbunden.

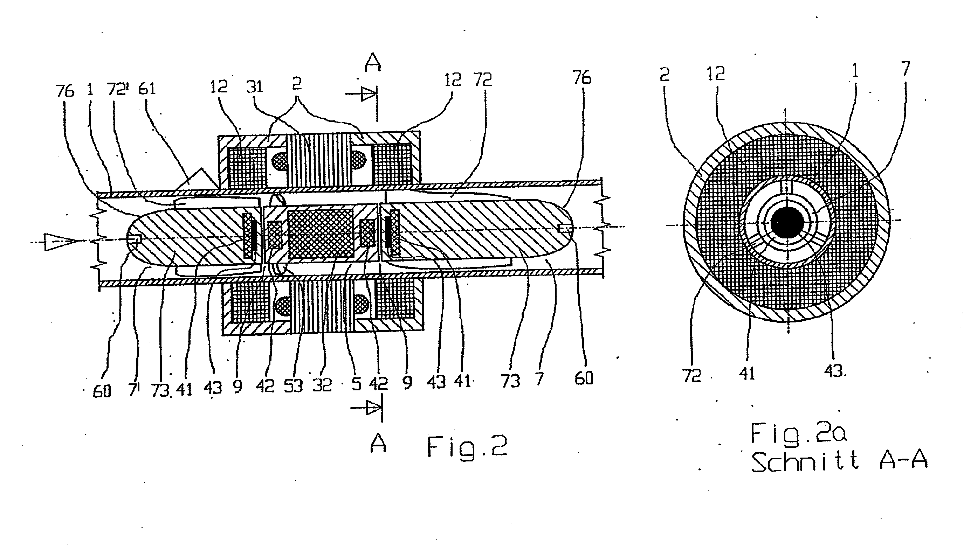

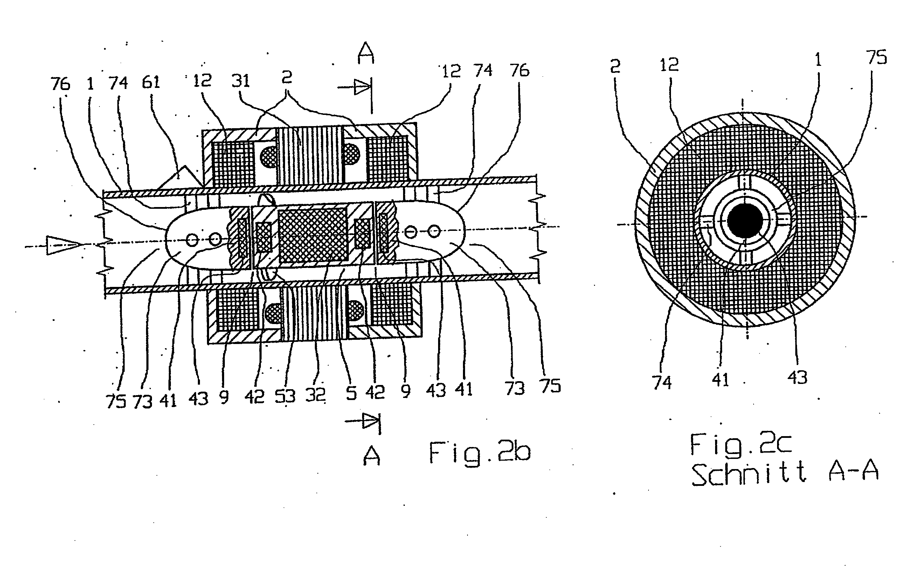

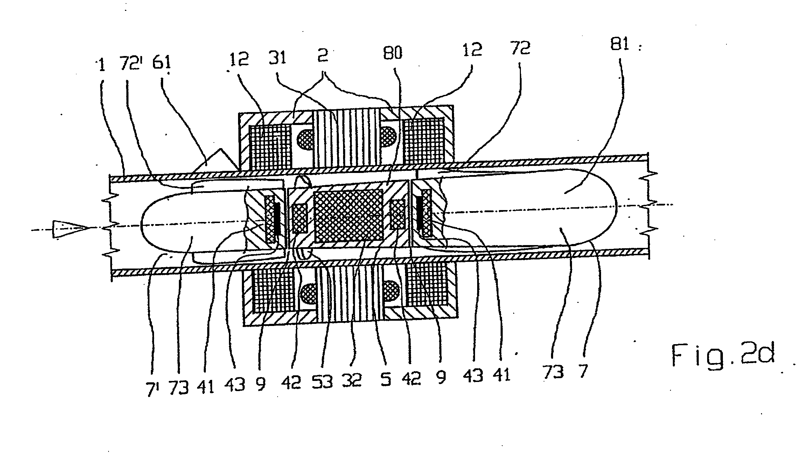

Die Funktion der magnetischen Lagerung ist anhand von Fig. 2 und 2a beschrieben.

Fig. 2 und Fig. 2a zeigen weiterhin jeweils im Längsschnitt und im Querschnitt eine weitere beispielhafte Ausführung einer Blutpumpe mit einer magnetisch gelagerten Rotornabe 52. In der Rotornabe 52 ist der Motorrotor 32 mit jeweils an den Enden angeordneten permanentmagnetischen Lagerelementen 42 kombiniert, die in einer Fassung 4 gehaltert sind. In den Fluid-Leiteinrichtungen 7 und 7' sind direkt permanentmagnetischen Lagerelementen 42 gegenüberstehend permanentmagnetische Lagerelemente 41 angeordnet. Die permanentmagnetischen Lagerelemente 41 und 42 sind entgegengesetzt gepolt. Die axial gerichtete, sich zwischen den permanentmagnetischen Lagerelementen 41 und 42 ausbildende Anziehungskraft sorgt dafür, dass das Förderteil 5 koaxial im rohrförmigen Hohlkörper 1 gehalten wird und radiale Auslenkungen korrigiert werden. Positionssensoren 43, die ebenfalls in den Fluid-Leiteinrichtungen 7 und 7' angeordnet sind, ermitteln die Breite des Nabenspaltes 9 und regeln diese über den Axialstabilisator 12. Der Axialstabilisator 12 ist in einem Stabilisatorgehäuse 2 angeordnet. Die Axialstabilisatoren 12, ausgebildet als Spulen, erzeugen bei eingeschaltetem Strom ein Magnetfeld, das über das Stabilisatorgehäuse 2 und die Flussleitstücke 10 so geleitet wird, dass das Förderteil 5 eine stabile axiale Lage zwischen den Fluid-Leiteinrichtungen 7 und 7' einnimmt. An den Enden der Fluid-Leiteinrichtungen 7 und 7' sowie an der Außenwand des rohrförmigen Hohlkörpers 1 sind Drucksensoren 60 sowie ein Flusssensor 61 zur Charakterisierung der Strömung angebracht. Das aus dem Motorrotor 32 und den permanentmagnetischen Lagerelementen 42 sowie der Rotorbeschaufelung 53 bestehende Förderteil 5 wird über den Motorstator 31 in Rotation versetzt. Radiale Abweichungen bei der Rotation werden durch die entgegengesetzt gepolten permanentmagnetischen Lagerelemente abgefangen, während die axiale Stabilisierung über die Positionssensoren 43 und die Axialstabilisatoren 12 erfolgt. Die Konzentrierung der Hauptmasse der permanentmagnetischen Lagerelemente 42 im Bereich der Achse des Förderteiles 5 ermöglicht, die Pumpe in einer pulsatilen Betriebsweise zu betreiben, beispielsweise durch schnelle Drehzahländerung des Rotors.

Alternativ sind die permanentmagnetischen Lagerelemente 41 und 42 statt als Vollzylinder als permanentmagnetische Ringe ebenfalls mit axialer Magnetisierung ausgebildet. Beliebige, dem Fachmann bekannte Ausgestaltungen können für die genaue Ausbildung der permanentmagnetischen Lagerelemente 41 und 42 verwendet werden.

Für eine Stabilisierung der axialen Lage des Förderteiles 5 bzw. der Rotornabe 52 ist ein Axialstabilisator 12 vorgesehen, der mit Positionssensoren 43 zusammenwirkt und der über die Fluid-Leiteinrichtungen 7 und 7' auf das Förderteil 5 jeweils stirnseitig einwirkt und eine hier nicht dargestellte elektronische Steuerschaltung benutzt. Der Axialstabilisator 12 bewirkt eine aktive Regelung der axialen Lage des Förderteiles 5, wobei die Stabilisierungsspulen entsprechend der vorgenommenen Regelung mit Strömen beaufschlagt werden und dabei einen axialen Magnetfluß erzeugen, der den axialen Magnetfluß der permanentmagnetischen Elemente überlagert und der Regelung der axialen Lage dient. Die Positionssensoren 43 stellen Abweichungen von der axialen Sollposition des Förderteiles 5 fest und leiten diese Information an die Steuerschaltung weiter.

Fig. 2b und Fig. 2c zeigen im Längs- und im Querschnitt eine weitere beispielhafte Ausführung einer erfindungsgemäßen Vorrichtung. Die Halterungen 75, die in Strömungsrichtung gesehen vor und hinter dem Förderteil 5 angeordnet sind, bestehen aus einer Nabe 73, die mit Stützen 74 an der Innenwand des rohrförmigen Hohlkörpers 1 befestigt sind. Die Stützen 74 sind hier beispielhaft im Abstand von 90° um die Nabe 73 angeordnet. Grundsätzlich würde auch eine Stütze 74 ausreichen. Die Halterung 75 dient im Wesentlichen der Aufnahme der permanentmagnetischen Lagerelemente 41. Für die axiale Stabilisierung sorgen der Axialstabilisator 12, der Positionssensor 43 und eine nicht dargestellte Regelelektronik.

In Fig. 2d sind in weiterer beispielhafter Ausführung das Förderteil 5 und die Fluid-Leiteinrichtung 7 konisch ausgebildet. Ein konischer Rotor 80 des Förderteiles 5 vergrößert sich in Strömungsrichtung und geht weiter konisch sich vergrößernd in eine konische Leiteinrichtung 81 über. Die permanentmagnetischen Lagerelemente 41 und 42 sind entgegengesetzt gepolt, die axiale Stabilisierung erfolgt auch hier über die Positionssensoren 43 in Verbindung mit dem Axialstabilisator 12.

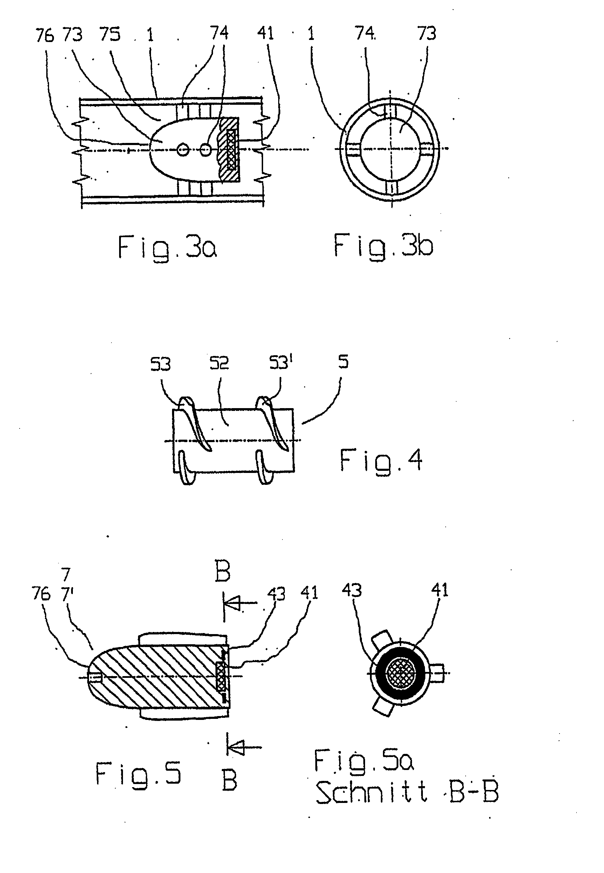

Die Figuren 3a und 3b zeigen jeweils im Längs- und im Querschnitt im Detail eine beispielhafte Ausführung der Halterung 75 mit Stützen 74.

Fig. 4 zeigt ein Förderteil 5 mit der Rotornabe 52, um die herum zwei Rotorbeschaufelungen 53 und 53' angeordnet sind. Die Anordnung von zwei oder mehr Rotorbeschaufelungen 53 ermöglicht es, die Wirkung der Beschaufelung des Förderteiles 5 zu erhöhen.

In Fig. 5 und Fig. 5a sind im Längs- und im Querschnitt Fluid-Leiteinrichtungen 7 bzw. 7' dargestellt, bei denen das permanentmagnetische Lagerelement 41 vom Positionssensor 43 umgeben ist.

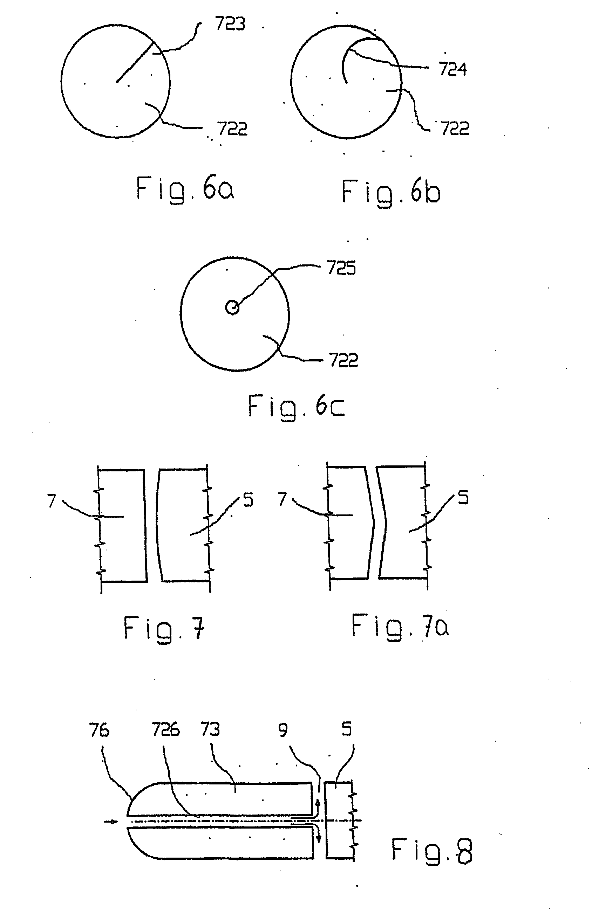

Maßnahmen, die die radiale Druckverteilung beeinflussen und Ausgleichsströmungen zur Verhinderung von Totwassergebieten im Bereich der Rotornabe 52, das heißt im Nabenspalt 9 zwischen den Stirnseiten der Fluid-Leiteinrichtung 7 und 7' und Förderteil 5, bewirken, sind in Fig. 6a, b, c, 7 und 7a dargestellt. In Fig. 6a ist auf einer Stirnseite 722 der Fluid-Leiteinrichtung 7, 7' eine sich vom Zentrum radial nach außen erstreckende Rippe 723 angeordnet.

In Fig. 6b ist die Rippe 724 gebogen ausgebildet. Statt derartiger Rippen können an der Stirnseite 722 auch konvexe und/oder konkave Wölbungen, radiale Beschaufelungen, Mikroschaufeln, Rippen, Rillen und exzentrische Erhöhungen 725 (Fig. 6c) beliebiger Form oder auch einfach eine Rauhigkeit der Oberfläche vorgesehen sein. Entscheidend ist allein, dass es sich hierbei um Mittel handelt, durch die das Fluid bei Rotation des Förderteiles 5 radial aus dem Nabenspalt 9 (vgl. Fig. 8) herausbefördert wird. Selbstverständlich können diese Mittel auch an der Stirnseite der Rotornabe 52 angeordnet sein.

Die Darstellung gemäß Fig. 7 bewirkt vorteilhafterweise zusätzlich eine Verbesserung der Notlaufeigenschaften im Falle des Ausfallens der Axialstabilisierung.

In Fig. 8 weist die Nabe 73 eine axiale Bohrung 726 auf, die vom zu fördernden Fluid durchströmt wird und bewirkt, dass im Nabenspalt 9 befindliches Fluid zusätzlich radial transportiert wird.

Es wird darauf hingewiesen, dass die erfindungsgemäße Magnetlagerung nicht auf zylindrische Formen der Magnete beschränkt ist. Weitere geometrische Ausgestaltungen der permanentmagnetischen Lagerelemente 41 und 42 sind möglich.

### Bezugszeichenliste

- 1: Rohrförmiger Hohlkörper
- 2: Stabilisatorgehäuse
- 3: Pumpengehäuse
- 4: Fassung
- 5: Förderteil
- 6: Krümmer
- 6': Krümmer
- 7: Fluid-Leiteinrichtung
- 7': Fluid-Leiteinrichtung
- 8: Rotorspalt
- 9: Nabenspalt
- 10: Flussleitstück
- 11: Kabelstutzen
- 11a: Kabel
- 12: Axialstabilisator
- 31: Motorstator
- 32: Motorrotor
- 41: permanentmagnetisches Lagerelement
- 42: permanentmagnetisches Lagerelement
- 43: Positionssensor
- 44 45 51 52: Rotornabe
- 53: Rotorbeschaufelung
- 60: Drucksensor
- 61: Flusssensor
- 62: Aortenkanüle
- 63: Verbindungselement
- 72: Fluid-Leitbeschaufelung
- 72': Fluid-Leitbeschaufelung
- 73: Nabe
- 74: Stütze
- 75: Halterung
- 76: Nabenkappe
- 722: Stirnseite
- 723: Rippe
- 724: Rippe
- 725: Erhöhung
- 726: Bohrung
- 80: konischer Rotor
- 81: konische Leiteinrichtung

## Patentansprüche

1. Vorrichtung zur schonenden Förderung von ein- oder mehrphasigen Fluiden, bestehend aus einem rohrförmigen, das Fluid im Wesentlichen axial führenden Hohlkörper (1), in dem in axialer Ausrichtung ein mit einem außerhalb des Hohlkörpers (1) befindlichen Motorstator (31) in Rotation versetzbares Förderteil (5) gelagert ist, wobei das Fluid im Rotorspalt (8) zwischen Förderteil (5) und Hohlkörper (19 förderbar ist, und vor und hinter dem Förderteil (5) je ein Befestigungselement (7, 7', 75) angeordnet ist, **dadurch gekennzeichnet, dass**
in dem vor dem Förderteil befindlichen Befestigungselement (7') ein Drucksensor (60) zur Druckmessung zuströmenden Fluids vorgesehen ist oder in beiden Befestigungselementen (7, 7') Sensoren (60) zur Erfassung der von der Pumpe erzeugten Druckdifferenz integriert sind.

2. Vorrichtung nach Anspruch 1, **dadurch gekennzeichnet, dass** das Förderteil (5) zwischen den Befestigungselementen (7, 7', 75), durch je einen Nabenspalt (9) getrennt, mittels sowohl in den Befestigungselementen (7, 7', 75) als auch im Förderteil (5) angeordneten, funktioneil zusammenwirkenden, sich mit ihren magnetisch wirksamen Flächen gegenüberstehenden, in axialer Richtung magnetisierten, entgegengesetzt gepolten permanentmagnetischen Lagerelementen (41, 42) berührungsfrei gelagert ist und Sensoren (43) und Stabilisatoren (12) zur Positionserfassung und Positionskorrektur des Förderteiles (5) in den Befestigungselementen (7, 7', 75) und an oder in der Wandung des Hohlkörpers (1) angeordnet sind.

3. Vorrichtung nach Anspruch 1 oder 2, **dadurch gekennzeichnet, dass** die Lagerelemente (41, 42) Flussleitstücke (10) aufweisen, die in den Befestigungselementen (7, 7', 75) angeordnet sind.

4. Vorrichtung nach einem der Ansprüche 1 bis 3,
**dadurch gekennzeichnet, dass** die Befestigungselemente (7, 7', 75) als Fluid-Leiteinrichtungen (7, 7') mit Fluidbeschaufelungen (72) ausgebildet sind.

5. Vorrichtung nach einem der Ansprüche 1 bis 4,
**dadurch gekennzeichnet, dass** an den dem Förderteil (5) zugewandten Stirnseiten (722, 723) der Befestigungselemente (7, 7', 75) und/oder an den Stirnseiten des Förderteiles (5) Rippen (723, 724) sowie Beschaufelungen, Rillen, konvexe und/oder konkave Ausbuchtungen oder exzentrisch angeordnete Erhöhungen (725) angebracht, sind.

6. Vorrichtung nach einem der Ansprüche 1 bis 5,
**dadurch gekennzeichnet, dass** in mindestens einem der Befestigungselemente (7, 7', 75) eine axial verlaufende Bohrung (726) angeordnet ist.

7. Vorrichtung nach einem der Ansprüche 1 bis 6,
**dadurch gekennzeichnet, dass** die Rotornabe (52) des Förderteiles (5) in axialem Abstand zwei Rotorbeschaufelungen (53) aufweist.

8. Vorrichtung nach einem der Ansprüche 1 bis 7,
**dadurch gekennzeichnet, dass** die Rotornabe (52) und die Naben (73) zylinderförmig ausgebildet und die Naben (73) durch Nabenkappen (76) an dem dem Förderteil (5) abgewandten Ende abgeschlossen sind.

9. Vorrichtung nach einem der Ansprüche 1 bis 8, **dadurch gekennzeichnet, dass** das Förderteil (5) und die Halterungen (75), auch in der Ausbildung als Fluid-Leiteinrichtungen (7, 7') in Strömungsrichtung nichtzylindrisch vergrößert oder verjüngt ausgebildet sind.

10. Vorrichtung nach einem der vorhergehenden Ansprüche, **dadurch gekennzeichnet, dass** an der Wandung des rohrförmigen Hohlkörpers ein Flusssensor (61) zur Charakterisierung der Strömung angebracht ist.

## Claims

1. Device for the gentle delivery of single- or multiphase fluids, consisting of a tubular hollow body (1), guiding the fluid essentially axially, in which a delivery element(5) which can be rotated by a motor stator (31) arranged outside the hollow body (1), is supported axially positioned, wherein the fluid is transportable in the rotor gap (8) between the delivery element (5) and the hollow body (19), and wherein one mounting element (7, 7', 75) is mounted in front and behind the delivery element (5), respectively,
**characterized in that**
a pressure sensor (60) for measuring the pressure of the inflowing fluid is provided in the mounting element (7') arranged in front of the delivery element or sensors (60) for detecting the pressure difference generated by the pump are integrated in both mounting elements (7, 7').

2. Device according to claim 1, **characterized in that** the delivery element (5) is supported free of contact between the mounting elements (7, 7', 75), separated each by a hub gap (9), by means of permanent magnetic bearing elements (41, 42) which are arranged in the mounting elements (7, 7', 75) as well as in the delivery element (5) and functionally work together and are opposingly with their magnetically acting feaces and which magnetic bearing elements (41, 42) are magnetised in the axial direction and are oppsitely poled and **in that** sensors (43) and stabilizers (12) for the determination of the position and the correction of the position of the delivery element (5) are arranged in the mounting elements (7, 7', 75) and on or in the wall of the hollow body (1).

3. Device according to claim 1 or 2, **characterized in that** the bearing elements (41, 42) have flow guide elements (10), which are arranged in the mounting elements (7, 7', 75).

4. Device according to one of claims 1 to 3, **characterized in that** the mounting elements (7, 7', 75) are formed as fluid guide units (7, 7') with fluid bladings (72).

5. Device according to one of claims 1 to 4, **characterized in that** on the end faces (722, 723) of the mounting elements (7, 7', 75) facing the delivery element (5) and/or on the end faces of the delivery element (5), ribs (723, 724) as well as bladings, grooves, convex and/or concave protuberances or eccentrically arranged projections (725) are provided.

6. Device according to one of claims 1 to 5, **characterized in that** an axially extending bore (726) is arranged in at least one of the mounting elements (7, 7', 75).

7. Device according to one of claims 1 to 6, **characterized in that** the rotor hub (52) of the delivery element (5) has two rotor bladings (53) arranged with axial distance.

8. Device according to one of claims 1 to 7, **characterized in that** the rotor hub (52) and the hubs (73) are formed cylindrically and that the hubs (73) at the end facing away from the delivery element (5) are closed by hub caps (76).

9. Device according to one of claims 1 to 8, **characterized in that** the delivery element (5) and the mountings (75), also when designed as fluid guide units (7, 7'), are formed non-cylindrically enlarged or diminished in the flow direction.

10. Device according to one of the preceding claims, **characterized in that** a flow sensor (61) for characterizing the flow is mounted to the wall of the tubular hollow body.

## Revendications

1. Dispositif destiné au conveyage précautionneux de fluides à une ou plusieurs phases, composé d'un corps creux (1) tubulaire, conduisant le fluide de façon essentiellement axiale, dans lequel est logée en sens axial une partie transporteuse (5) pouvant être amenée en rotation avec un stator moteur (31) situé à l'extérieur du corps creux (1), le fluide pouvant être transporté dans l'interstice du rotor (8) entre la partie transporteuse (5) et le corps creux (1), et un élément de fixation (7, 7', 75) étant disposé respectivement devant et derrière la partie transporteuse (5),
**caractérisé en ce que**
un capteur de pression (60) pour la mesure de pression du fluide amené est agencé dans l'élément de fixation (7') disposé davant la partie transporteuse ou des capteurs (60) pour détecter la différence de pression généree par la pompe sont intégrés dans les deux éléments de fixation (7, 7').

2. Dispositif selon revendication 1, **caractérisé en ce que** la partie transporteuse (5) entre les éléments de fixation (7, 7', 75) est séparée à chaque fois par un interstice de moyeu (9) et est logée sans contact au moyen d'éléments palier (41, 42) à magnétisme permanent, à polarisation inverse, magnétisés en sens axial et don't les surfaces magnétiquement efficaces se font face, coopérant d'un point de vue fonctionnel et disposés aussi bien dans les éléments de fixation (7, 7', 75) que dans la partie transporteuse (5), et **en ce que** des capteurs (43) et des stabilisateurs (12) destinés à détecter et corriger la position de la partie transporteuse (5) sont disposés dans les éléments de fixation (7, 7', 75) et sur ou dans la paroi du corps creux (1).

3. Dispositif selon la revendication 1 ou 2, **caractérisé en ce que** les éléments paliers (41, 42) présentent des éléments de guidage du flux (10) qui sont disposés dans les éléments de fixation (7, 7', 75).

4. Dispositif selon L# une des revendications 1 à 3, **caractérisé en ce que** les éléments de fixation (7, 7', 75) sont configurés comme des arrangements de guidage du fluide (7, 7') avec un agencement d'ailettes pour fluide (72).

5. Dispositif selon l'une des revendiactions 1 à 4, **caractérisé en ce que** des nervures (723, 724) ainsi que un agencement d'ailettes, rainures, courbures convexes et/ou concaves ou des élévations (725) disposées de façon excentrique sont pratiquées sur les faces frontales (722, 723) des éléments de fixation (7, 7', 75) tournées vers la partie transporteuse (5) et/ou sur les faces frontales de la partie transporteuse (5).

6. Dispositif selon l'une des revendiations 1 à 5, **caractérisé en ce qu'**un alésage (726) de sens axial est prévu dans au moins un des éléments de fixation (7, 7', 75).

7. Dispositif selon l'une des revendications 1 à 6, **caractérisé en ce que** le moyeu du rotor (52) de la partie transporteuse (5) présente à distance axiale deux ailettages de rotor (53).

8. Dispositif selon l'une des revendiations 1 à 7, **caractérisé en ce que** le moyeu du rotor (52) et les moyeux (73) sont configurés en forme de cylindre et **en ce qu'**à leur extrémité située à l'opposé de la partie transporteuse (5), les moyeux (73) se terminent par des couvres-moyeux (76).

9. Dispositif selon l'une des revendications 1 à 8, **caractérisé en ce que** la partie transporteuse (5) et les fixations (75) présentent dans l'axe d'écoulement une forme augmentée ou rétrécie de manière non cylindrique, ce égalemen lorsqu#elles sont configurées comme arryangements de guidage de fluide (7, 7').

10. Dispositif selon l'une des revendications précédente, **caractérisé en ce que** un capteur de flux (61) pour la caractérisation de l'écoulement est disposé sur la paroi du corps creux.
